# FASCICULE DE BREVET EUROPEEN

(11) **EP 2 470 896 B1**
(45) Date de publication et mention de la délivrance du brevet: **24.06.2015**
(21) Numéro de dépôt: 10752872.1
(22) Date de dépôt: 27.07.2010
(51) Int. Cl.: G01N 33/20, C22C 14/00

(54) **PROCEDE POUR LA DETECTION DE LA CONTAMINATION D'ALLIAGES DE TITANE DE TYPE BIPHASE AVEC UNE PHASE ALPHA ET UNE PHASE BETA**
VERFAHREN ZUR ERKENNUNG VON KONTAMINATION BEI ZWEIPHASIGEN TITANLEGIERUNGEN MIT EINER ALPHA-PHASE UND EINER BETA-PHASE
METHOD FOR DETECTING THE CONTAMINATION OF TWO-PHASE TITANIUM ALLOYS WITH AN ALPHA PHASE AND A BETA PHASE

(30) Priorité: 28.08.2009 FR 0955866
(43) Date de publication de la demande: 04.07.2012
(73) Titulaire: Snecma, 75015 Paris (FR)
(72) Inventeur: BERTHOD, Gilles, F-91300 Massy (FR)
(74) Mandataire: Berbinau, Pierre Jean Marie
(86) Numéro de dépôt international: PCT/FR2010/051583
(87) Numéro de publication internationale: WO 2011/023874

(56) Documents cités:
- US-A- 4 551 434
- US-A- 5 039 612
- US-A- 5 413 649
- US-A1- 2007 137 734

## Description

La présente invention concerne un procédé d'examen d'un alliage de titane de type biphasé avec une phase alpha et une phase béta.

La phase alpha désigne une des phases présentes dans la plupart des alliages de titane (Ti), et correspond à un réseau cristallin hexagonal compact des atomes de Ti.

Les alliages de titane comportant une phase alpha sont facilement contaminés par d'autres éléments chimiques avec lesquels ils sont en contact. Par exemple, ils sont contaminés par des gaz (tels que oxygène, azote, hydrogène, halogènes). Pour des raisons de cinétique chimique cette contamination est habituellement visible lorsque le matériau a été exposé à une température au voisinage de 500°C ou plus. Cette contamination entraîne une fragilisation de l'alliage de titane depuis la surface exposée, qui se traduit par une détérioration de ses caractéristiques mécaniques.

Pour cette raison, les traitements thermiques qu'un alliage de titane subit lors de sa fabrication sont effectués sous vide, c'est-à-dire avec une exposition aux gaz suffisamment faible pour que la surface de l'alliage de titane ne soit pas contaminée.

En dépit de ces précautions, une contamination de la surface de l'alliage peut se produire. Il est donc indispensable de vérifier la présence ou l'absence de contamination. Plusieurs techniques de détection de contamination de surface sont actuellement utilisées.

Une première technique de détection est l'analyse chimique de l'alliage. Cette analyse chimique s'effectue, de façon connue, au moyen d'une microsonde. Cette technique est onéreuse et peu fiable, et qualitative (elle ne permet pas une mesure de l'étendue de la contamination).

Une seconde technique est l'essai mécanique. Par exemple on utilise, de façon connue, une éprouvette de traction entaillée de cet alliage que l'on teste jusqu'à rupture. Cette technique est onéreuse, peu fiable, et qualitative. Alternativement, on peut utiliser, de façon connue, une tôle fine de cet alliage que l'on plie jusqu'à l'apparition de fissures. Cette technique est seulement qualitative.

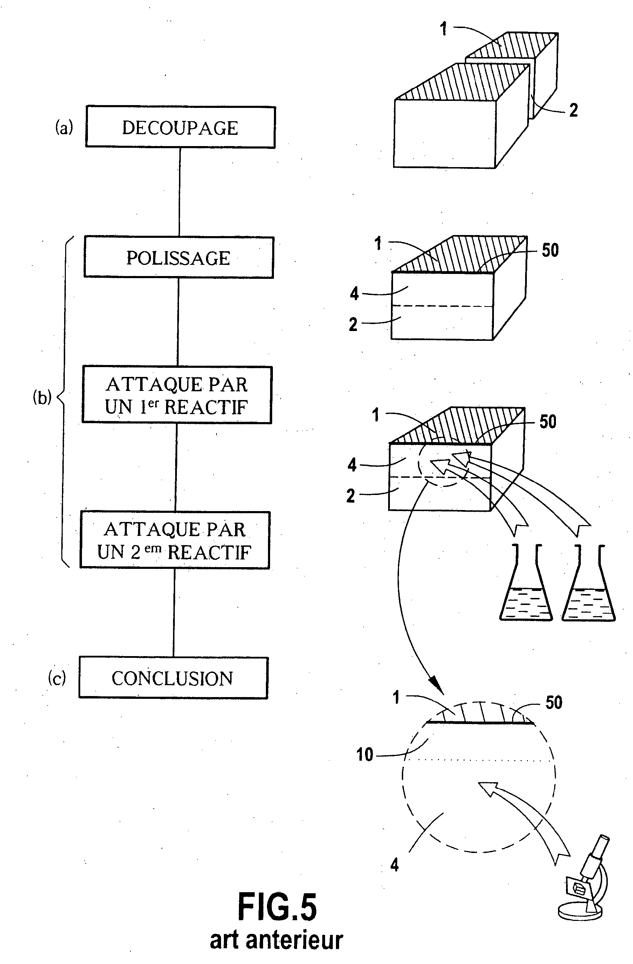

Une troisième technique, illustrée par le document US4551434, est l'examen de la microstructure de l'alliage de titane. Les étapes de cette technique connue sont illustrées schématiquement en figure 5. On découpe un échantillon d'une pièce en cet alliage (étape (a)) de telle sorte que la surface de découpe 2 débouche sur la surface extérieure 1 de la pièce. Puis on effectue un polissage d'une région 4 de cette surface de découpe 2, cette région 4 étant située au voisinage du bord 50 de cet échantillon, ce bord 50 étant en commun avec la surface extérieure 1 de la pièce, et on applique ensuite sur cette région 4 successivement un premier réactif chimique, puis un second réactif chimique (étape (b)). Ces attaques chimiques par ces réactifs ont pour but de révéler la microstructure de l'alliage. On observe ensuite le bord de l'échantillon au microscope optique pour y déceler la présence ou l'absence d'un liseré blanc 10 (étape (c)).

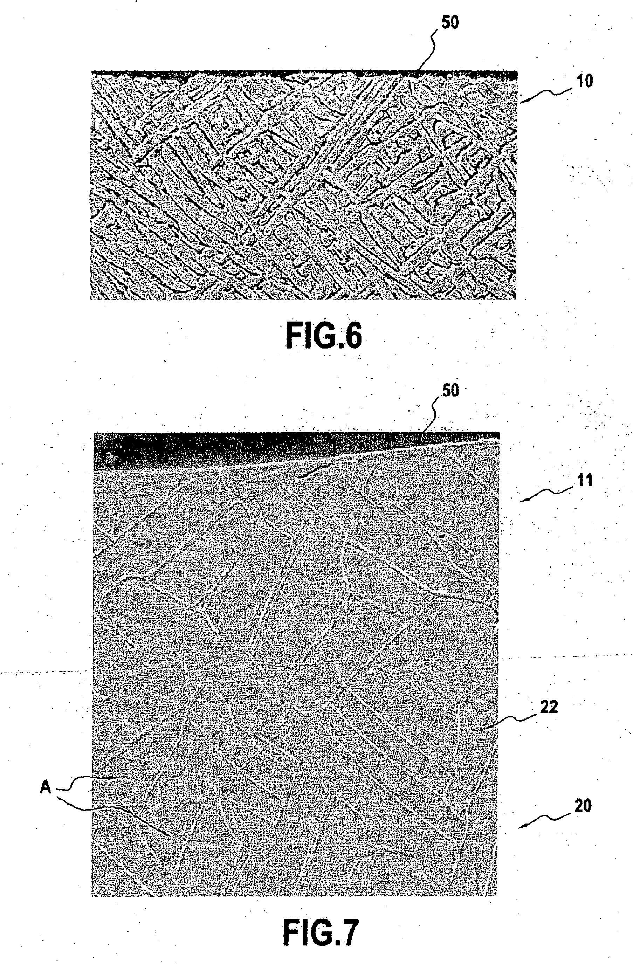

La figure 6 est une microphotographie au microscope optique d'une surface de découpe d'une pièce en alliage de titane TA6Zr4DE contaminé à l'oxygène, avec un grossissement de ×500. On note la présence d'un liseré blanc 10 le long du bord 50 de l'échantillon. Il est connu qu'un tel liseré blanc 10 est le signe d'une contamination de l'alliage par des gaz à partir de sa surface. La profondeur de la contamination est donnée par l'épaisseur de ce liseré blanc 10.

Cependant cette technique d'examen métallographique reste parfois peu précise. En effet, la détection de la contamination, fondée seulement sur l'appréciation visuelle d'un contraste entre le liseré blanc et les parties plus grises adjacentes, et la taille variable des grains, ne permettent pas une mesure précise de l'épaisseur du liseré blanc, donc cette technique ne permet pas dans tous les cas de connaître précisément l'étendue de la contamination.

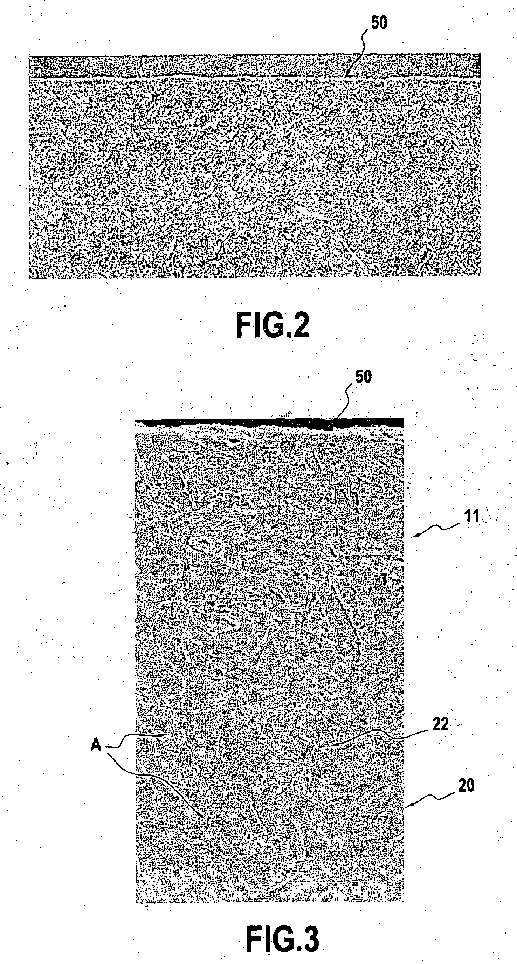

De plus, cette technique n'est pas applicable à certains alliages de titane tels que le TA5CD4. Ainsi, sur la figure 2, qui est une microphotographie au microscope optique d'une surface de découpe d'un alliage de titane TA5CD4 contaminé à l'oxygène, on n'observe pas de liseré blanc le long du bord 50 de l'échantillon.

La présente invention vise à remédier à ces inconvénients.

L'invention vise à proposer un procédé qui permette de déterminer si un alliage de titane est contaminé par des éléments chimiques gazeux étrangers, qui s'applique à tous les alliages de titane de type biphasé avec une phase alpha et une phase béta, et qui permette une mesure plus précise de cette contamination.

Ce but est atteint grâce au fait que ce procédé comporte les étapes suivantes :
(a) On découpe un échantillon d'une pièce en ledit alliage,
(b) On prépare une région de la surface de découpe de l'échantillon située au voisinage du bord de cet échantillon, ce bord étant en commun avec la surface extérieure de la pièce, de façon à permettre l'observation de cette région,
(c) On observe la phase alpha de cette région, à un grossissement supérieur à ×5000,
(d) On décide de la présence ou de l'absence de granulosités dans la phase alpha d'une première zone contigüe à ce bord de l'échantillon,
(e) On conclut à l'existence d'une contamination de l'alliage par un gaz si on constate une absence de granulosités dans la phase alpha de cette zone contigüe alors que des granulosités sont présentes dans la phase alpha hors de cette zone contigüe.

Grâce à ces dispositions, il est possible de déterminer de façon fiable si un alliage de titane de type biphasé avec une phase alpha et une phase béta a été contaminé par des éléments chimiques gazeux étrangers, quelque soit cet alliage de titane. De plus, le grossissement supérieur auquel l'observation est réalisée permet une mesure précise de cette contamination, car la frontière entre une zone sans granulosités et une zone avec granulosités est ainsi bien définie.

Avantageusement, la préparation de la région de l'échantillon d'alliage de titane comprend un polissage de cette région puis une attaque chimique de cette région avec un réactif unique.

Ainsi, il n'est plus nécessaire, pour préparer la surface de l'échantillon d'alliage de titane, d'utiliser deux réactifs. L'examen d'un échantillon est donc plus simple et plus fiable.

L'invention sera bien comprise et ses avantages apparaîtront mieux, à la lecture de la description détaillée qui suit, d'un mode de réalisation représenté à titre d'exemple non limitatif. La description se réfère aux dessins annexés sur lesquels :
- la figure 1 est une représentation schématique des étapes du procédé selon l'invention,
- la figure 2 est une microphotographie au microscope optique d'une surface de découpe d'un alliage de titane TA5CD4 contaminé à l'oxygène,
- la figure 3 est une microphotographie au microscope électronique à balayage de la surface de découpe de l'alliage de titane TA5CD4 de la figure 2, à un grossissement supérieur,
- la figure 4 est une représentation schématique de la microstructure observée en figure 3,
- la figure 5 est une représentation schématique des étapes du procédé d'examen, selon l'art antérieur, de la microstructure d'un alliage de titane,
- la figure 6 est une microphotographie au microscope optique d'une surface de découpe d'un alliage de titane TA6Zr4DE contaminé à l'oxygène, avec un grossissement de ×500,
- la figure 7 est une microphotographie au microscope électronique à balayage de la surface de découpe de l'alliage de titane TA6Zr4DE de la figure 6, à un grossissement supérieur,
- la figure 8 est une représentation schématique de la microstructure observée en figure 7.

Jusqu'à présent, si dans une pièce en alliage de titane de type biphasé avec une phase alpha et une phase béta, on n'observait pas de liseré blanc le long du bord de l'échantillon en commun avec la surface de cette pièce, on en concluait que cette pièce n'avait pas été contaminée. Donc, si les performances mécaniques de cette pièce n'étaient pas satisfaisantes, on en concluait que ces performances inférieures étaient le résultat par exemple d'un défaut de fabrication, d'un mauvais état de surface, d'un écrouissage, d'une mauvaise condition de fonctionnement. En effet, chacun de ces évènements peut expliquer des performances mécaniques inférieures.

Les inventeurs ont rassemblé un grand nombre d'échantillons de divers alliages de titane de type biphasé avec une phase alpha et une phase béta, et ont pensé, de façon non-évidente, à observer ces échantillons à un grossissement bien supérieur au grossissement habituel d'environ ×500 qui est suffisant pour observer les liserés blancs des alliages contaminés en surface par des éléments gazeux. Ainsi, avec un grossissement égal ou supérieur à ×5000, les inventeurs ont remarqué, de façon inattendue, que certaines zones de phase alpha ne présentaient pas de granulosités tandis que d'autres zones de phase alpha en présentaient.

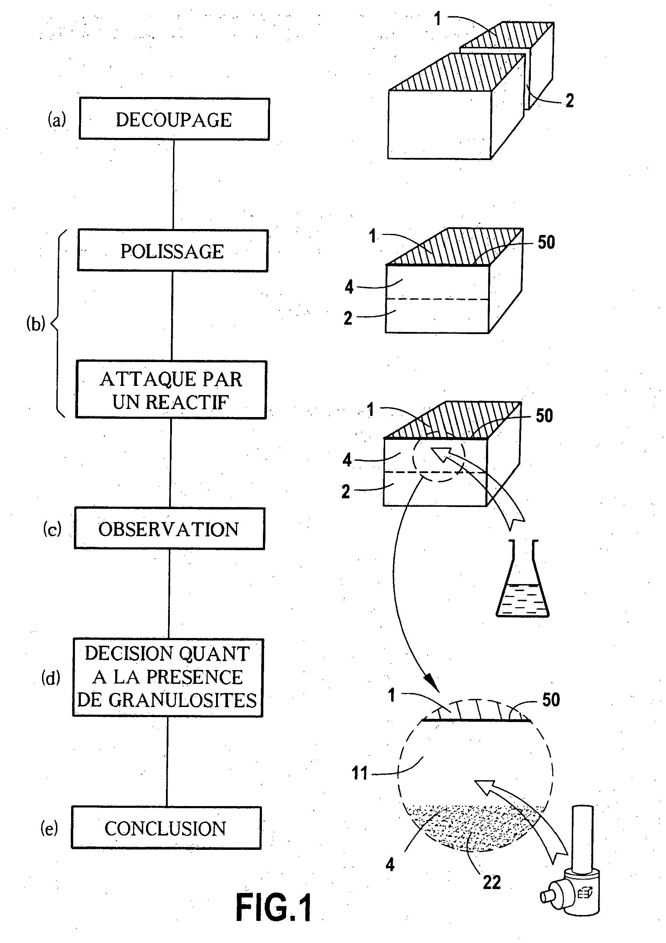

La figure 1 est une représentation schématique des étapes du procédé selon l'invention, qui rend possible l'observation des granulosités.

Tout d'abord on découpe un échantillon d'une pièce en alliage de titane de type biphasé avec une phase alpha et une phase béta (étape (a)) de telle sorte que la surface de découpe 2 débouche sur la surface extérieure 1 de la pièce.

On prépare ensuite une région 4 de la surface de découpe 2, cette région 4 étant située au voisinage du bord 50 de cet échantillon (étape (b)), ce bord 50 étant en commun avec la surface extérieure 1 de la pièce. Cette préparation a pour but de permettre l'observation de cette région 4.

Par exemple, cette préparation comprend un polissage de cette région 4 puis une attaque chimique de cette région 4 avec un réactif unique. En effet, contrairement au procédé selon l'art antérieur dans lequel il est nécessaire d'utiliser deux réactifs en succession, et pendant des durées différentes, il est possible dans le procédé selon l'invention d'utiliser un seul réactif. Il en résulte une simplification du procédé et une atténuation du risque d'une mauvaise préparation.

Par exemple, le polissage est un polissage spéculaire.

Par exemple, ce réactif est une solution aqueuse d'acide fluorhydrique HF à 0,5%. Ce réactif est appliqué sur la surface de l'échantillon pendant une durée comprise entre 15 secondes et 30 secondes.

Alternativement, il est possible d'utiliser plus d'un réactif.

On observe ensuite la phase alpha de la région, à un grossissement au moins égal à x5000 (étape (c)).

Ces observations sont effectuées avec un microscope électronique à balayage (MEB).

Alternativement, ces observations peuvent être réalisées avec un autre microscope permettant un grossissement supérieur à ×5000. Cependant ces observations ne peuvent être effectuées avec un microscope optique actuel, dont le grossissement maximum est environ d'un millier de fois.

Par exemple, ce grossissement est supérieur à ×10000.

On décide alors de la présence ou de l'absence de granulosités dans la phase alpha de la zone contigüe 11 au bord de l'échantillon (étape (d)).

Puis on conclut à l'existence d'une contamination de l'alliage par un gaz si on constate une absence de granulosités dans la phase alpha de ladite zone contigüe 11 alors que des granulosités 22 sont présentes dans la phase alpha hors de ladite zone contigüe 11 (étape (e)).

Ainsi, comme le montre la figure 7, qui est une microphotographie au MEB à un grossissement de x5000 de la surface de découpe d'un échantillon de l'alliage de titane TA6Zr4DE contaminé à l'oxygène dont une microphotographie au microscope optique est également présenté en figure 6, on constate que dans une première zone 11 contigüe au bord 50 de l'échantillon la phase alpha A ne comporte pas de granulosités, tandis que dans une seconde zone 20 plus éloignée du bord 50, des granulosités 22 sont présentes au sein de la phase alpha A.

Ainsi, les inventeurs ont observé une absence de granulosités dans la première zone 11 contigüe, et qui correspond au liseré blanc 10 observé sur la figure 6.

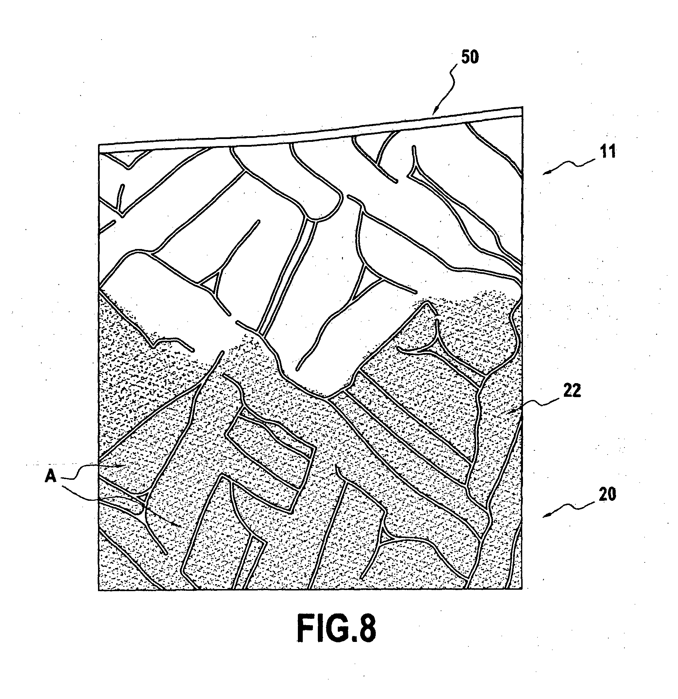

La figure 8 illustre schématiquement la structure observée sur la figure 7.

Afin de confirmer l'hypothèse selon laquelle l'absence de granulosités 22 dans la phase alpha de la zone contigüe 11 au bord de l'échantillon est corrélée avec une contamination (de cette zone contigüe 11) de cet échantillon avec un gaz, les inventeurs ont observé le bord d'alliages de titane TA6Zr4DE non-contaminés mais ayant subi une modification en surface (par exemple un écrouissage, un polissage). Les inventeurs ont constaté la présence de granulosités 22 dans la phase alpha de la zone contigue 11 au bord 50 d'une pièce en un de ces alliages, ce qui valide l'hypothèse ci-dessus.

Avantageusement, le procédé selon l'invention permet de déterminer si un alliage de titane TA5CD4 a été contaminé en surface ou pas, alors que cette information n'est pas possible avec un procédé d'observation selon l'art antérieur. Ainsi, la figure 3 est une microphotographie au MEB à un grossissement de x5000 de la surface de découpe de l'alliage de titane TA5CD4 dont une microphotographie au microscope optique est également présentée en figure 2. On constate que dans une première zone contigüe 11 au bord 50 de l'échantillon la phase alpha A ne comporte pas de granulosités, tandis que dans une seconde zone 20 plus éloignée du bord 50 (c'est-à-dire une zone hors de la zone contigüe 11), des granulosités 22 sont présentes au sein de la phase alpha A.

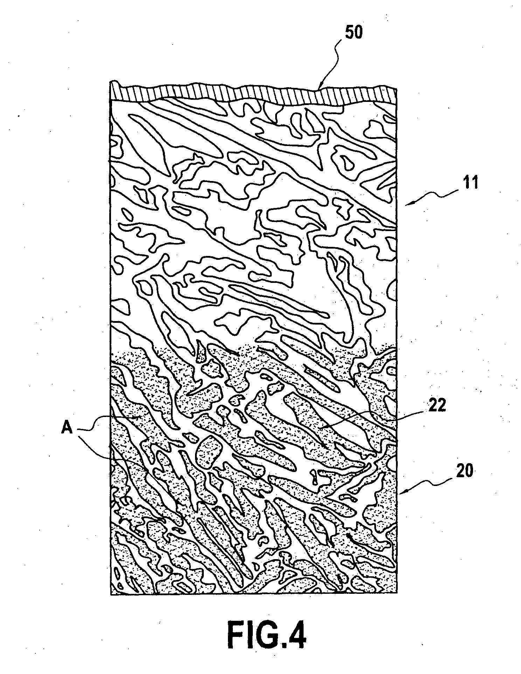

La figure 4 illustre schématiquement la structure observée sur la figure 3.

## Revendications

1. Procédé d'examen d'un alliage de titane de type biphasé avec une phase alpha et une phase béta, **caractérisé en ce qu'**il comporte les étapes suivantes :
(a) On découpe un échantillon d'une pièce en ledit alliage,
(b) On prépare une région (4) de la surface de découpe dudit échantillon située au voisinage du bord (50) de cet échantillon, ce bord (50) étant en commun avec la surface extérieure (1) de la pièce, de façon à permettre l'observation de ladite région (4),
(c) On observe la phase alpha de ladite région (4), à un grossissement supérieur à ×5000,
(d) On décide de la présence ou de l'absence de granulosités dans la phase alpha d'une première zone (11) contigüe audit bord (50) de l'échantillon,
(e) On conclut à l'existence d'une contamination dudit alliage par un gaz si on constate une absence de granulosités dans la phase alpha de ladite zone contigüe (11) alors que des granulosités (22) sont présentes dans la phase alpha hors de ladite zone contigüe (11).

2. Procédé d'examen d'un alliage de titane selon la revendication 1, **caractérisé en ce que** ladite observation à l'étape (c) est effectuée avec un microscope électronique à balayage.

3. Procédé d'examen d'un alliage de titane selon la revendication 1 ou 2, **caractérisé en ce que** la préparation de ladite région (4) à l'étape (b) comprend un polissage de cette région (4) puis une attaque chimique de cette région (4) avec au moins un réactif.

4. Procédé d'examen d'un alliage de titane selon la revendication 3, **caractérisé en ce que** ladite attaque chimique est effectuée avec un réactif unique.

5. Procédé d'examen d'un alliage de titane selon la revendication 4, **caractérisé en ce que** le dit réactif est une solution aqueuse d'acide fluorhydrique HF à 0,5%.

## Patentansprüche

1. Verfahren zur Untersuchung einer Titanlegierung vom zweiphasigen Typ mit einer Alpha-Phase und einer Beta-Phase, **dadurch gekennzeichnet, dass** es die folgenden Schritte umfasst:
(a) aus einem Stück aus der Legierung wird eine Probe ausgeschnitten,
(b) ein Bereich (4) der Schnittfläche der Probe, welcher in der Nähe des Randes (50) dieser Probe gelegen ist, wird präpariert, wobei dieser Rand (50) mit der Außenfläche (1) des Stücks gemein ist, um die Betrachtung des Bereichs (4) zu ermöglichen,
(c) die Alpha-Phase des Bereichs (4) wird mit einer mehr als 5000fachen Vergrößerung betrachtet,
(d) es wird über das Vorliegen oder Nichtvorliegen von Körnigkeiten in der Alpha-Phase eines an den Rand (50) der Probe angrenzenden ersten Bereichs (11) entschieden,
(e) es wird auf das Vorhandensein einer Kontamination der Legierung durch ein Gas geschlossen, wenn ein Nichtvorliegen von Körnigkeiten in der Alpha-Phase des angrenzenden Bereichs (11) festgestellt wird, während Körnigkeiten (22) in der Alpha-Phase außerhalb des angrenzenden Bereichs (11) vorhanden sind.

2. Verfahren zur Untersuchung einer Titanlegierung nach Anspruch 1, **dadurch gekennzeichnet, dass** die Betrachtung bei Schritt (c) mit einem Rasterelektronenmikroskop vollzogen wird.

3. Verfahren zur Untersuchung einer Titanlegierung nach Anspruch 1 oder 2, **dadurch gekennzeichnet, dass** das Präparieren des Bereichs (4) bei Schritt (b) ein Polieren dieses Bereichs (4), anschließend ein chemisches Ätzen dieses Bereichs (4) mit wenigstens einem Reagenz umfasst.

4. Verfahren zur Untersuchung einer Titanlegierung nach Anspruch 3, **dadurch gekennzeichnet, dass** das chemische Ätzen mit einem einzigen Reagenz durchgeführt wird.

5. Verfahren zur Untersuchung einer Titanlegierung nach Anspruch 4, **dadurch gekennzeichnet, dass** das Reagenz eine 0,5%ige wässrige Flusssäure HF-Lösung ist.

## Claims

1. A method of examining a titanium alloy of two-phase type with an alpha phase and a beta phase, the method being **characterized in that** it comprises the following steps:
(a) cutting a sample of a part made of said alloy;
(b) preparing a region (4) of the cut surface of said sample that is situated in the vicinity of the edge (50) of said sample, said edge (50) being in common with the outside surface (1) of the part, so as to enable said region (4) to be observed;
(c) observing the alpha phase of said region (4) at a magnification of greater than x5000;
(d) deciding on whether granularity is present or absent in the alpha phase of a first zone (11) contiguous with said edge (50) of the sample; and
(e) concluding that said alloy has been contaminated with a gas if granularity is found to be absent in the alpha phase of said contiguous zone (11) whereas granularity (22) is present in the alpha phase outside said contiguous zone (11).

2. A method of examining a titanium alloy according to claim 1, **characterized in that** said observation in step (c) is performed with a scanning electron microscope.

3. A method of examining a titanium alloy according to claim 1 or claim 2, **characterized in that** the preparation of said region (4) in step (b) comprises polishing said region (4) and then chemically etching said region (4) with at least one reagent.

4. A method of examining a titanium alloy according to claim 3, **characterized in that** said chemical etching is performed using a single reagent.

5. A method of examining a titanium alloy according to claim 4, **characterized in that** said reagent is an aqueous solution of hydrofluoric acid HF at 0.5%.
